# EUROPEAN PATENT SPECIFICATION

(11) **EP 0 700 285 B1**
(45) Date of publication and mention of the grant of the patent: **23.09.1998**
(21) Application number: 94916318.2
(22) Date of filing: 27.05.1994
(51) Int. Cl.: A61K 9/48, A61K 9/50, A61K 31/505

(54) **A DRUG DELIVERY COMPOSITION FOR ALPHA-ADRENO RECEPTOR BLOCKING AGENTS**
ARZNEISTOFFABGABEZUSAMMENSETZUNGEN FÜR ALPHA-ADRENORGISCHEN REZEPTORBLOCKIERENDEN AGENTEN
COMPOSITION MEDICAMENTEUSE A BASE D'AGENT BLOQUANT DU RECEPTEUR ALPHA-ADRENERGIQUE S'ADMINISTRANT ORALEMENT

(30) Priority: 29.05.1993 GB 9311191
(43) Date of publication of application: 13.03.1996
(73) Proprietor: SYNTHELABO, 92350 Le Plessis Robinson (FR)
(72) Inventor: ILLUM, Lisbeth, The Park, Nottingham NG7 1BA (GB); WATTS, Peter, West Bridgford, Nottingham NG2 6DR (GB); FARRAJ, Nidal, West Bridgford, Nottingham NG2 7TS (GB)
(74) Representative: McNeeney, Stephen Phillip
(86) International application number: GB9401158
(87) International publication number: WO9427582

(56) References cited:
- EP-A- 0 036 350
- EP-A- 0 339 811
- EP-A- 0 348 808
- GB-A- 2 036 558
- THERAPIE, vol.48, no.2, April 1993, PARIS pages 115 - 118 FOURTILLAN J.B. ET AL 'PHARMACOKINETICS OF PRAZOSIN ADMINISTERED AS GASTRO-INTESTINAL-THERAPEUTIC-SYSTEMS TO 24 HEALTHY VOLUNTEERS'

## Description

The present invention relates to drug delivery composition comprising alfuzosin or prazosin, and more particularly to such a composition which is adapted for once-daily administration.

Alpha-adreno receptor blocking agents can be beneficial in the treatment of hypertension, congestive heart failure and Raynaud's syndrome. They are particularly beneficial in the treatment of benign hypertrophy. Prazosin and alfuzosin are examples of alpha-adreno receptor blocking agents.

Alfuzosin is a quinazoline derivative which acts as a selective and competitive antagonist of alpha-adreno receptor mediated contraction of prostatic, prostatic capsule, bladder base and proximal urethral structures. It thereby reduces the tone of these structures and has a place in the treatment of symptoms of benign prostatic hyperplasia.

A review of the pharmacodynamic and pharmacokinetic properties has been given by Wilde *et al*. Drugs 435 410 (1993). The drug is presently given at a dose of 7.5 to 10mg per day in two or three divided doses.

Alfuzosin is a weak base with a pKa of 8.1. The partition coefficient of the drug between octanol and water can be predicted from a computer package such as C-LOG P version 3.54 (Craig, P.N. (1990) Drug Compendium. In: Hansch, C., Sammes, P.G., Taylor, J.B. (eds) Comprehensive Medicinal Chemistry. Vol. 6: Cumulative Subject Index and drug Compendium. Pergamon Press, New York, pp 237-991). The predicted value ranging from 10 to 80 indicates that the drug will most likely be less than completely absorbed from the colonic region of the gastrointestinal tract, as discussed in the work of Taylor *et al*. (Models for Intestinal Permeability to Drugs, Chapter 11, in: Drug Delivery to the Gastrointestinal Tract, editors Hardy *et al*. Ellis Horwood, Chichester, 1989. The mean oral bioavailability for the immediate release dosage form of alfuzosin is 64% and absorption is reported to be unaffected by concomitant food intake. The volume of distribution is 2.5L/kg and it is 90% protein bound. The plasma elimination half life ranges from 3 to 6 (mean 4.8) hours after intravenous administration. The mean half life after oral administration is also 4.8 hours. Alfuzosin is extensively metabolised by the liver with only 11% of the parent compound excreted unchanged in the urine. The metabolites are inactive. Other α-adreno receptor blocking drugs such as prazosin are expected to have similar physicochemical properties to alfusozin.

Many drugs are currently administered in so-called "once-daily" formulations which only need to be administered once every twenty four hours. Such once-daily formulations are favoured by both patients and doctors because they are more convenient and lead to better patient control and patient compliance. Examples of drugs currently administered in once-daily formulations are nifedipine, oxprenolol, propranolol, carbamazepine, theophylline, indomethacin and diltiazem.

The currently known once-daily formulations are typically controlled release formations which give a fairly constant sustained release of the drug following administration. However, such a release profile would be unsuitable for α-adreno receptor blocking agents such as alfuzosin or prazosin. In order to achieve a good therapeutic effect using alfuzosin it is necessary to maintain a reasonably steady plasma profile. However, with the current once-daily formulations the plasma level of alfuzosin will drop significantly once the formulation reaches the colonic region due to the poor absorption of the drug from this region. Furthermore, this problem cannot be overcome by increasing the amount of drug released as the amount of drug absorbed before the colonic region would be too high, and high peaks in the plasma profile of alfuzosin are associated with adverse side effects.

We have now found that an improved composition of alfuzosin or prazosin for once-daily administration can be achieved by providing a composition which provides a biphasic drug release profile. The invention therefore provides an oral drug delivery composition comprising alfuzosin or prazosin characterised in that the composition is adapted to release the drug in two portions, the first portion of the drug being released in the upper gastrointestinal tract and the second portion of the drug being released by sustained release in the terminal ileum and/or the colon. This biphasic release profile should provide the required plasma levels of the drug, especially once the composition has reached the colonic region, whilst minimising the onset of adverse side-effects such as postural hypotension, associated with too rapid release of the drug.

The composition is conveniently provided in at least two units, for example tablets, pellets or microcapsules. These units may be administered separately but substantially simultaneously and may be provided in a pack which dispenses or indicates the correct units to be taken each day. Alternatively the units may be enclosed within a single capsule, such as a gelatin capsule, for release substantially immediately after administration.

At least one of the units is adapted not to release the drug, the second portion, until it reaches the terminal ileum or the colon, hereinafter referred to as the "colonic region". This delayed drug release may be achieved using systems known in the art for colonic delivery, such as timed delay release systems, or coatings which are only dissolved by the conditions present in the colonic region. Preferred coatings are pH-sensitive coatings which dissolve at pH 5 or above, or redox sensitive coatings as are known to those in this art.

The second portion of the drug should be released in a sustained manner and to achieve this, the unit(s) containing the second portion should also comprise a controlled release system. Controlled release formulations known in the art can be used, and hydrophilic gel matrices or wax matrices are preferred.

Preferably the unit(s) containing the first portion of the drug also release the drug in a sustained manner and may therefore comprise a controlled release formulation as for the second portion of the drug.

The total amount of drug present in a single dose of the composition will typically be between 8mg and 15mg, more preferably 9mg to 12mg, and a dose of 10mg is most preferred. The amount of the total drug released as the first portion may be equal to that released as the second portion, but it is preferred that the second portion of the drug released is larger than the first portion. A preferred ratio of first portion to second portion is 3:7.

By providing that the second portion of drug which is released is larger than the first portion, a sufficiently high concentration gradient can be obtained to provide acceptable absorption of the drug in the colon.

Pulsatile systems comprising distinct doses of drug delivered to the upper and lower intestines have been described by Mehta *et al*. in US 4728512 and 4794001 and by Abramowitz *et al*. in US 5158777. However, for alfuzosin at least the second portion should not be delivered as a pulsatile or fast bolus administration since this could lead to adverse reactions associated with a high peak plasma concentration.

The formulations of the invention can be used to treat the same conditions as prior art formulations of α-adreno receptor blocking agents are used for, for example, hypertension, pheochromocytoma, shock and peripheral vascular diseases (for example, Raynauds's syndrome and acrocyanosis).

The invention will now be described in detail with reference to the following drawings in which:
Figure 1 shows two suitable biphasic drug release profiles for a composition according to the invention;
Figure 2 shows the release of alfuzosin HCl from a sustained-release tablet formulation with 3mg alfuzosin per tablet and 0.1M HCl dissolution medium;
Figure 3 shows release of alfuzosin HCl from a coated sustained-release tablet formulation, 3mg per tablet; and
Figure 4 shows the release of alfuzosin HCl from a composition comprising one uncoated tablet as in Figure 2 and two coated tablets as in Figure 3.

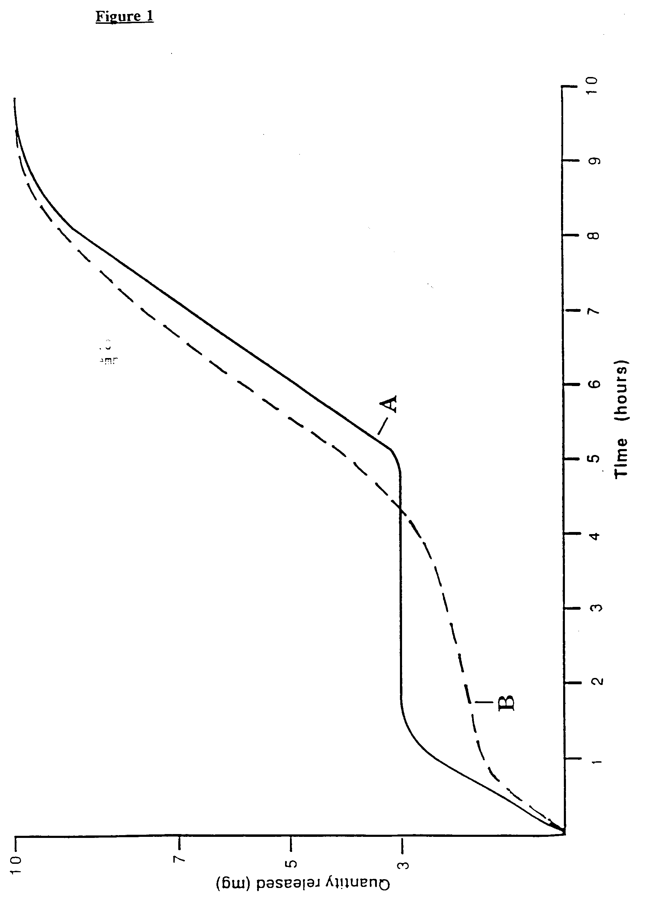

The present invention is based on the discovery that an improved once-daily formulation of alfuzosin or prazosin can be achieved if it provides a biphasic drug release profile. Figure 1 shows two optimum profiles which have been produced by computer modelling analysis of alfuzosin absorption from the gastrointestinal tract based upon the method originally described by Farraj *et al*. ("Microcomputer simulation of the *in vivo* performance of matrix embedded drugs following oral administration", Proc. Third European Congress of Biopharmaceutics and Pharmacokinetics, Freiburg, F.I.P., 143, 1987). Such a release profile enables the plasma levels of alfuzosin required for therapeutic effect to be maintained without risking a high peak in plasma levels, which is associated with adverse side effects. This profile is achieved by providing a composition which is adapted to release a first portion of the drug in the upper gastrointestinal tract and to release the second portion of the drug in a sustained manner in the colonic region. The two portions of the drug may be released such that they are separated by a period of time where no release of drug occurs, thus giving profile A of Figure 1, or where the start of the release of the second portion overlaps with the end of the release of the first portion, giving profile B in Figure 1.

The release of the first portion of the drug, giving the first phase of the release profile may also take place in a sustained manner to further reduce the risk of side-effects. It is preferred that the release of the first portion of the drug starts substantially immediately after administration and should take place over a period of between 30 minutes and 6 hours following administration, but preferably over a period of 3 hours following administration.

The release of the second portion of the drug, giving the second phase of the release profile, should take place in a sustained manner, preferably over a period of between one hour before and 6 hours after the first phase, more preferably over a period of three hours after the first phase.

To provide reliable and uniform gastrointestinal transit each unit of the composition should conveniently be less than 12mm in size, preferably less than 10mm and more preferably less than 7mm.

The units used for releasing the second portion of the drug and optionally also for the first part may comprise any known controlled release system. Such continuous release systems have been described in various text books on pharmaceutical formulations, for example Gupta and Robinson "Oral controlled release delivery", Chapter 6 in: Treatise on Controlled Drug Delivery, Editor A. Kydonieus, Dekker, New York, 1992. A preferred control release composition is a controlled release matrix comprising a wax or a hydrophilic gel material such a hydroxypropyl methyl cellulose. In such hydrophilic gel matrices, the hydrophilic gum swells in contact with water and forms a gel layer which retards dissolution. The layer is also subject to erosion but the main release mechanism appears to be diffusion. Other controlled release systems include the osmotic pump system which consists of an osmotically active core, including the drug, surrounded by a rate controlling, semipermeable membrane with an orifice of controlled size. Rate controlled drug delivery depends on two factors; water permeability of the semipermeable membrane and the osmotic pressure of the core formulation. An ion-exchange resin complex may be used in which the drug is bound to an ion-exchange resin and coated with polymers, or the drug may be dispersed in a slowly eroding material such as a wax or fatty acid. Also a porous inert matrix tablet may be used in which the drug is distributed randomly within a porous inert matrix, a three dimensional network is formed through the pores of which the digestive juices and the dissolved drug can diffuse. The release of the drug depends on factors such as the porosity of the matrix, the solubility of the drug as well as pore length. The unit may be coated with a diffusion-controlling membrane in which release of the drug is controlled by diffusion through a non-disintegrating film.

The release of the drug from the unit or units containing the second portion of the drug can be delayed until the unit reaches the colonic region by any method known for colonic delivery of drugs. It is known that small intestine transit in man is approximately 3 hours (Davis *et al*. Gut **27** 886, 1986). It is therefore desirable that the start of the release of the second portion occurs from 3 to 5 hours after the formulation has left the stomach and has found its way to the colonic region.

The second unit may comprise a timed-delay release mechanism. The Time Clock Release System™ (Pozzi *et al*. APV Course on Pulsatile Drug Delivery, Konigswinter, 20 May 1992) is a tablet system where a tablet core containing the active drug is coated with a layer of pharmaceutical excipients. The excipients hydrate causing the surface layer to burst at a set time. The Pulsincap™ system is an oral pulsatile delivery system which may be configured to release its drug content at a predetermined time or place within the gastrointestinal tract. The device essentially consists of an impermeable capsule body which contains the drug, sealed at the neck orifice with a hydrogel plug. A normal gelatin cap is then placed onto the body of the device. After ingestion the gelatin cap dissolves allowing the plug to hydrate. At a predetermined and controlled time the swollen plug is ejected from the body of the device, thereby releasing the capsule contents and enabling the drug to be released. (Wilding *et al*., Pharm. Res. 9, 654, 1992 and Binns e*t al*., 3rd Eur. Symp. Control. Drug Del., Abstract Book, 1994, p124).

Another system which may be used is the time controlled explosion system as in US 4871549 (incorporated herein by reference).

Alternatively a coating which is sensitive to the specific conditions existing in the colon may be provided on the appropriate units. The colonic region has a high presence of microbial anaerobic organisms providing reducing conditions. Thus the coating may be redox-sensitive. Such coatings may comprise azopolymers which can for example consist of a random copolymer of styrene and hydroxyethyl methacrylate, cross-linked with divinylazobenzene synthesized by free radical polymerization. The azopolymer is broken down enzymatically and specifically in the colon or disulphide polymers (see PCT/BE91/00006 and Van den Mooter, Int. J. Pharm. 87, 37, 1992).

An Oros-CT™ system may be used which is similar to the osmotic pump mentioned above but has an enteric coating which dissolves in the colon. Other materials which dissolve in the colon are amylose (Milojevic *et al*., Proc. Int. Symp. Contr. Rel. Bioact. Mater. 20, 288, 1993), calcium pectinate (Rubenstein *et al*., Pharm. Res., 10, 258, 1993), chondroitin sulphate (Rubenstein *et al*., Phar. Res. 9, 276, 1992) and resistant starches (Allwood *et al*., PCT WO 89/11269, 1989), dextran hydrogels (Hovgaard and Brøndsted, 3rd Eur. Symp. Control. Drug Del., Abstract Book, 1994, 87) and pH-sensitive hydrogels (Kopecek *et al*., J. Control. Rel. 19, 121, 1992). Resistant starches, eg glassy amylose, are starches that are not broken down by the enzymes in the upper gastrointestinal tract but are degraded in the colon.

The thickness of the coating material should preferably be in the range of 80 to 300 µm, preferably 150-250 µm.

Preferred coating materials are those which dissolve at a pH of 5 or above. Such a coating can be made from a variety of polymers such as cellulose acetate trimellitate (CAT), hydroxypropylmethyl cellulose phthalate (HPMCP), polyvinyl acetate phthalate (PVAP), cellulose acetate phthalate (CAP) and shellac as described by Healy in his article "Enteric Coatings and Delayed Release" Chapter 7 in Drug Delivery to the Gastrointestinal Tract, editors Hardy et al., Ellis Horwood, Chichester, 1989. Especially preferred materials are methylmethacrylates or copolymers of methacrylic acid and methylmethacrylate. Such materials are available as Eudragit polymers (trademark) (Rohm Pharma, Darmstadt, Germany). Eudragits are copolymers of methacrylic acid and methylmethacrylate. Eudragit L100 dissolves at pH 6 and upwards and comprises 48.3% methacrylic acid units per g dry substance; Eudragit S100 dissolves at pH 7 and upwards and comprises 29.2% methacrylic acid units per g dry substance. Preferred coating compositions are based on Eudragit L100 and Eudragit S100 in the range 100 parts L100:0 parts S100 to 20 parts L100:80 parts S100. The most preferable range is 70 parts L100:30 parts S100 to 80 parts L100:20 parts S100. As the pH at which the coating begins to dissolve increases, the thickness necessary to achieve colon specific delivery decreases. For formulations where the ratio of Eudragit L100:S100 is high, a coat thickness of the order 150-200 µm is preferable. For coatings where the ratio Eudragit L100:S100 is low, a coat thickness of the order 80-120 µm is preferable.

The coatings may be applied by standard methods such as spray application using a fluid bed coater or rotary pan.

Specific embodiments will now be described with reference to the following examples.

### Example 1

A controlled release system was devised comprising three 6mm tablets, each containing 3mg of alfuzosin hydrochloride. Two of the three tablets contained an enteric coating, designed to delay drug release until the terminal ileum or the colonic region is reached. The three tablets were designed to fit inside a size O hard gelatin capsule.

Thirty tablets were prepared of the following composition:
- Alfuzosin hydrochloride: 3.0mg
- Hydroxypropylmethyl cellulose (Methocel K100M): 15.0mg
- Microcrystalline cellulose (Avicel): 23.0mg
- Dicalcium phosphate (Emcompress): 107.5mg
- Magnesium stearate: 1.5mg

Sufficient quantities of the powder components to prepare 30 tablets were blended together using a mechanical blender.

Round, biconvex tablets with a diameter of 6mm, were hand pressed from the powder blend using a Manesty F3 tablet press.

An enteric coating was applied to twenty of the alfuzosin tablets as follows. 39g of Eudragit L100 (methacrylic acid copolymer Type A, USP/Nf) and 13g of Eudragit S100 (Methacrylic acid copolymer Type B, USP,Nf) were dissolved in a mixture of 750ml of isopropanol and 20ml of water. 10g of dibutyl phthalate was mixed into the Eudragit solution as plasticizer. Finally, 10g of talc was mixed into the Eudragit solution. Twenty alfuzosin tablets and 450g of placebo tablets were placed into the coating chamber of an Aeromatic STREA-1 fluid bed coater. A coating of 200 µm in thickness was applied to the alfuzosin tablets.

The dissolution performance of the uncoated and enteric-coated alfuzosin tablets was assessed using USP Method II (paddles rotating at 50rpm). For the uncoated tablets, the dissolution test medium was 0.1M hydrochloric acid. For the coated tablets, the first 2h of the test were run in 0.1M hydrochloric acid. After 2h, the test medium was changed to 0.05M phosphate buffer, pH 7.2. Samples were withdrawn from the dissolution vessels at regular intervals and the appearance of alfuzosin hydrochloride was monitored spectrophotometrically (λₘₐₓ-246nm).

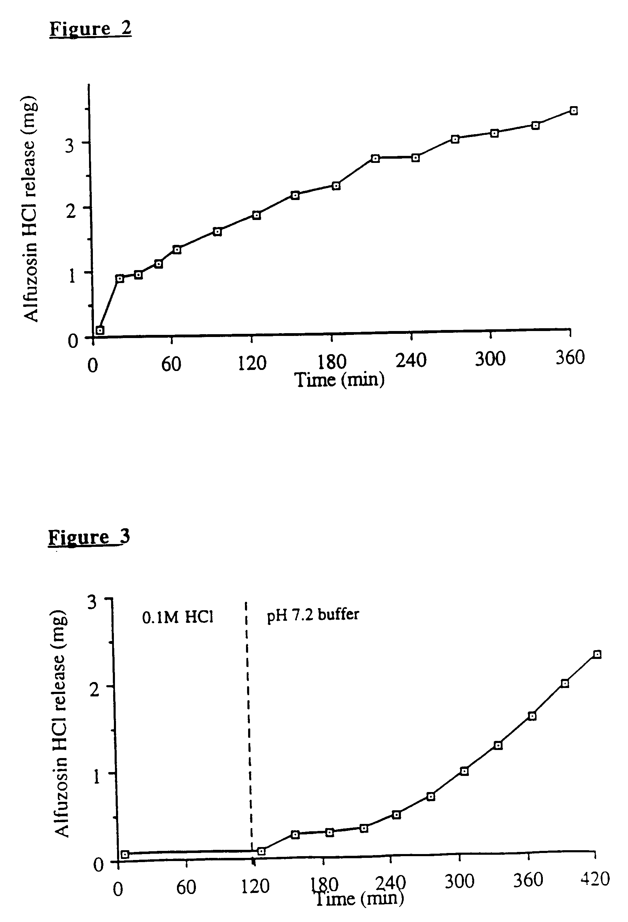

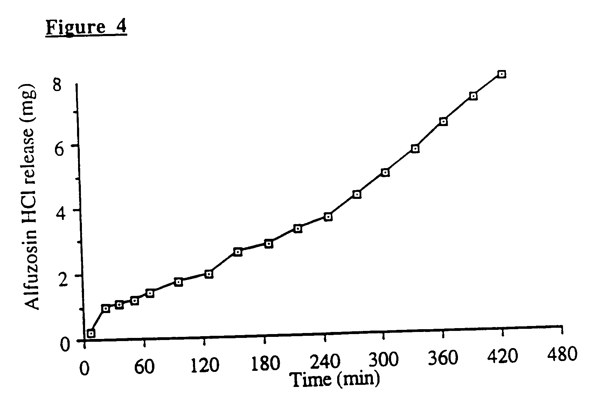

The dissolution performance of the uncoated controlled release tablets, which provide the first phase of release, is presented in Figure 2. The dissolution performance of the coated controlled release tablets, which provide the second phase, is presented in Figure 3. The required composite release profile representing a formulation comprising 1 uncoated and 2 coated tablets is presented in Figure 4.

Analysis of the release data indicated a release rate constant of 0.0096mg/min for the first phase of the controlled release profile (release from one uncoated tablet). For the second phase of the controlled release profile (release from 2 coated tablets), the rate constant was 0.024mg/min. Therefore this formulation provided an initial phase of slow drug release from the uncoated tablet followed by a more rapid total rate of release (by a factor of two) after 240 minutes as the enteric coating dissolved from the two coated tablets. The faster rate of drug release after 240 minutes will compensate for impaired drug absorption in the lower portion of the intestine and allow therapeutic plasma levels of drug to be maintained.

### Example 2

The uncoated controlled release tablets of Example 1 may be replaced by simple uncoated immediate release tablets of the following composition:
- Alfuzosin hydrochloride: 3.0mg
- Microcrystalline cellulose (Avicel): 38.0mg
- Dicalcium phosphate (Emcompress): 107.5mg
- Magnesium stearate: 1.5mg

The tablets may be round, biconvex in shape with a diameter of 6mm.

### Example 3

A tablet system consisting of an immediate controlled release tablet containing 3mg of alfuzosine and a colonic controlled release tablet containing 7mg of alfuzosine was prepared. The controlled matrix system consisted of 10% hydroxypropylcellulose (grade K100) mixed with 90% Avicel (microcrystalline cellulose). The tablets were compressed on a Manesty F3 fitted with 6.5mm concave punches. This system gave an *in vitro* controlled release profile where the drug was released over a 2 hour period and where 90% of the drug was released after 90 mins. Release studies were performed using the US Pharmacopoeia type 2 dissolution apparatus at 100rpm stirring speed in pH 6.8 phosphate buffer at 37°C. The colon targeting tablet system was produced by coating controlled release matrix tablet cores using the Aeromatic STREA-1 fluid bed coater. A solution of Eudragit L100 and Eudragit S in the ratio of 75:25 in isopropanol was prepared using talc as an anti-tack agent and dibutyl phthalate as plasticiser. The tablets of batch size 100 tablets were provided with a coating that represented a 19% weight gain of the tablets. The dissolution performance of the tablets was assessed by placing them for 2 hours in 0.1M hydrochloric acid followed by phosphate buffer at pH 6.8 buffer. After 2 hours in 0.1M HCl less than 5% of the drug was released. When transferred to pH 6.8 buffer, significant drug release only began after a 3 hour delay and release was 90% complete within 1.5 hours.

## Claims

1. An oral drug delivery composition comprising alfuzosin or prazosin characterised in that the composition is adapted to release the drug in two portions the first portion of the drug being released in the upper gastrointestinal tract and the second portion of the drug being released by sustained release in the terminal ileum and/or the colon.

2. The drug delivery composition according to claim 1 comprising at least two units wherein at least one unit contains the second portion of the drug and is coated with a coating material which is dissolved by the conditions in the terminal ileum, and/or the colon.

3. A drug delivery composition according to claim 2 wherein coating material is a pH-sensitive polymer or polymer mixture which dissolves at pH 5 or above.

4. A drug delivery composition according to claim 3 wherein the pH sensitive polymer is a copolymer of methacrylic acid and methyl methacrylate.

5. A drug delivery composition according to claim 2 wherein the coating material is a redox-sensitive polymer.

6. A drug delivery composition according to claim 1 wherein the composition comprises at least two units, at least one unit containing the second portion of the drug and comprises a timed-delay release mechanism.

7. A drug delivery composition according to any one of the preceding claims wherein the first portion of the drug is also released in a sustained manner.

8. A drug delivery composition according to claim 7 wherein the composition comprises a hydrophilic gel matrix or a wax matrix.

9. Use of a drug chosen from the group consisting of alfuzosin and prazosin for the production of an oral drug delivery composition for the treatment of benign prostatic hyperplasia, said oral drug delivery composition being adapted to release said drug in two portions, the first portion of the drug being released in the upper gastrointestinal tract and the second portion of the drug being released by sustained release in the terminal ileum and/or the colon.

## Patentansprüche

1. Orale Arzneimittelabgabezusammensetzung bzw. -zubereitung mit Alfuzosin oder Prazosin, dadurch gekennzeichnet, daß sie für eine Freisetzung des Arzneimittels in zwei Portionen ausgelegt ist, wobei die erste Portion des Arzneimittels im oberen Magen-Darm-Bereich freigesetzt und die zweite Portion des Arzneimittels durch verzögerte Freigabe im terminalen Ileum und/oder im Kolon freigesetzt werden.

2. Arzneimittelabgabezusammensetzung bzw. -zubereitung nach Anspruch 1, umfassend mindestens zwei Einheiten, von denen mindestens eine die zweite Portion des Arzneimittels enthält und mit einem Beschichtungsmaterial beschichtet ist, das unter den im terminalen Ileum und/oder im Kolon herrschenden Bedingungen in Lösung geht.

3. Arzneimittelabgabezusammensetzung bzw. -zubereitung nach Anspruch 2, wobei das Beschichtungsmaterial aus einem pH-empfindlichen Polymer oder Polymergemisch, das bei einem pH-Wert von 5 oder darüber in Lösung geht, besteht.

4. Arzneimittelabgabezusammensetzung bzw. -zubereitung nach Anspruch 3, wobei es sich bei dem pH-empfindlichen Polymer um ein Copolymer aus Methacrylsäure und Methylmethacrylat handelt.

5. Arzneimittelabgabezusammensetzung bzw. -zubereitung nach Anspruch 2, wobei es sich bei dem Beschichtungsmaterial um ein redoxempfindliches Polymer handelt.

6. Arzneimittelabgabezusammensetzung bzw. -zubereitung nach Anspruch 1, wobei die Zusammensetzung bzw. Zubereitung mindestens zwei Einheiten umfaßt und mindestens eine Einheit die zweite Portion des Arzneimittels enthält und einen zeitverzögerten Freigabemechanismus umfaßt.

7. Arzneimittelabgabezusammensetzung bzw. -zubereitung nach einem der vorhergehenden Ansprüche, wobei die erste Portion des Arzneimittels auch verzögert freigesetzt wird.

8. Arzneimittelabgabezusammensetzung bzw. -zubereitung nach Anspruch 7, wobei die Zusammensetzung bzw. Zubereitung eine hydrophile Gelmatrix oder eine Wachsmatrix umfaßt.

9. Verwendung eines Arzneimittels aus der Gruppe Alfuzosin und Prazosin zur Herstellung einer oralen Arzneimittelabgabezusammensetzung bzw. -zubereitung zur Behandlung von gutartigem Prostatakrebs, wobei die orale Arzneimittelabgabezusammensetzung bzw. -zubereitung zur Freisetzung des Arzneimittels in zwei Portionen ausgelegt ist und die erste Portion des Arzneimittels im oberen Magen-Darm-Bereich und die zweite Portion des Arzneimittels verzögert im terminalen Ileum und/oder im Kolon freigesetzt werden.

## Revendications

1. Composition pour l'administration orale de médicament comprenant de l'alfuzosine ou de la prazosine, caractérisée en ce que la composition est adaptée pour libérer le médicament en deux portions, la première portion du médicament étant libérée dans l'appareil gastro-intestinal supérieur et la seconde portion du médicament étant libérée par libération prolongée, dans l'iléon terminal et/ou le colon.

2. Composition pour l'administration de médicament selon la revendication 1 comprenant au moins deux unités, dans laquelle au moins une unité contient la seconde portion du médicament et est enrobée par un matériau d'enrobage qui est dissous par les conditions existant dans l'iléon terminal et/ou le colon.

3. Composition pour l'administration de médicament selon la revendication 2, dans laquelle le matériau d'enrobage est un polymère ou un mélange de polymère sensible au pH qui se dissous à pH 5 ou plus.

4. Composition pour l'administration de médicament selon la revendication 3, dans laquelle le polymère sensible au pH est un copolymère d'acide méthacrylique et de méthacrylate de méthyle.

5. Composition pour l'administration de médicament selon la revendication 2, dans laquelle le matériau d'enrobage est un polymère sensible à l'oxydoréduction.

6. Composition pour l'administration de médicament selon la revendication 1, dans laquelle la composition comprend au moins deux unités, au moins une unité contenant la seconde portion du médicament et comprend un mécanisme de libération retardée.

7. Composition pour l'administration de médicament selon l'une quelconque des revendications précédentes, dans laquelle la première portion du médicament est également libérée d'une manière prolongée.

8. Composition pour l'administration de médicament selon la revendication 7, dans laquelle la composition comprend une matrice de gel hydrophile ou une matrice de cire.

9. Utilisation d'un médicament choisi parmi le groupe constitué de l'alfuzosine et de la prazosine pour la production d'une composition pour l'administration orale de médicament pour le traitement de l'hyperplasie prostatique bénigne, ladite composition pour l'administration orale de médicament étant adaptée pour libérer ledit médicament en deux portions, la première portion du médicament étant libérée dans l'appareil gastro-intestinal supérieur et la seconde portion du médicament étant libérée par libération prolongée, dans l'iléon terminal et/ou le colon.
